# EUROPÄISCHE PATENTSCHRIFT

(11) **EP 1 850 897 B1**
(45) Veröffentlichungstag und Bekanntmachung des Hinweises auf die Patenterteilung: **23.05.2018**
(21) Anmeldenummer: 06706240.6
(22) Anmeldetag: 16.01.2006
(51) Int. Cl.: A61M 11/00

(54) **INHALATIONSTHERAPIEVORRICHTUNG UND VERFAHREN ZU DEREN BETRIEB**
INHALATION TREATMENT DEVICE AND METHOD FOR THE OPERATION THEREOF
DISPOSITIF DE TRAITEMENT PAR INHALATION ET PROCEDE POUR FAIRE FONCTIONNER CE DERNIER

(30) Priorität: 11.02.2005 DE 102005006372
(43) Veröffentlichungstag der Anmeldung: 07.11.2007
(73) Patentinhaber: PARI Pharma GmbH, 82319 Starnberg (DE)
(72) Erfinder: BORGSCHULTE, Markus, 81675 München (DE); ACHTZEHNER, Wolfgang, 82239 Alling (DE)
(74) Vertreter: Hoffmann Eitle
(86) Internationale Anmeldenummer: PCT/EP2006/000315
(87) Internationale Veröffentlichungsnummer: WO 2006/084543

(56) Entgegenhaltungen:
- WO-A-02/17988
- WO-A-2004/028606
- GB-A- 2 262 452
- US-A- 5 551 416

## Beschreibung

Die Erfindung betrifft Inhalationstherapievorrichtungen mit einem Aerosolerzeuger, der aus der Inhalationstherapievorrichtung entnehmbar bzw. in diese einsetzbar ist, sowie ein Verfahren zu deren Betrieb.

GB 2 262 452 A beschreibt eine Inhalationsvorrichtung mit einem Medikamentenreservoir, das eine Information bezüglich des Medikaments umfasst. Diese Information kann durch die Inhalationsvorrichtung detektiert werden.

WO 2004/028606 A beschreibt ein Aerosoltherapiegerät in Verbindung mit einem Persönlichen Digitalen Assistenten. Das Aerosoltherapiegerät umfasst verschiedene Sensoren, u.a. zum Erfassen eines Zustands des Membran-Aerosolerzeugers, beispielsweise dessen Temperatur und Stromaufnahme, den Füllstand des Flüssigkeitsreservoirs oder eine andere therapierelevante Größe des Aerosolerzeugers.

WO 02/17988 A beschreibt eine Medikamenten-Cartridge mit einem Informationsspeicherelement zum Speichern einer Information bzgl. des Medikaments sowie eines Medikamentenabgabeprotokolls.

Mit Inhalationstherapievorrichtungen werden Aerosole für therapeutische Zwecke erzeugt, die hohen Anforderungen gerecht werden müssen. Dementsprechend hoch ist der qualitätssichernde Aufwand bei der Konstruktion und der Fertigung dieser Vorrichtungen. Verschiedene Faktoren nehmen aber während der Benutzung der Vorrichtung durch einen Patienten Einfluss auf die Funktionsfähigkeit und Dosisgenauigkeit der Inhalationstherapievorrichtung.

Vor diesem Hintergrund hat die Erfindung zum Ziel, Maßnahmen anzugeben, mit denen die Funktionsfähigkeit und Dosisgenauigkeit einer Inhalationstherapievorrichtung sichergestellt werden kann.

Das Ziel wird erfindungsgemäß erreicht, durch eine Vorrichtung mit den Merkmalen des Anspruchs 1 und ein Verfahren nach Anspruch 20. Vorteilhafte Ausgestaltungen ergeben sich aus den Unteransprüchen.

Die Erfindung geht dabei von der Vorstellung aus, dass das Kernstück einer Inhalationstherapievorrichtung der hier in Rede stehenden Art der Aerosolerzeuger ist, der in der Inhalationstherapievorrichtung angeordnet ist und der aus einem in Form einer Flüssigkeit vorliegenden Medikament ein Aerosol erzeugt, wenn er von einer in der Inhalationsvorrichtung vorhandenen Steuereinheit zur Aerosolerzeugung angesteuert wird. Der Aerosolerzeuger weist typischerweise zumindest eine Membran und einen Schwingungserzeuger auf, wobei die Membran von dem Schwingungserzeuger in Schwingungen versetzt werden kann und dann aus der auf die eine Seite der Membran zugeführten Flüssigkeit ein Aerosol erzeugt. Insbesondere in hochwertigen Inhalationstherapievorrichtungen ist der Aerosolerzeuger aus der Vorrichtung entnehmbar, um beispielweise gründlich gereinigt, gegen einen Aerosolerzeuger anderer Bauart ausgetauscht oder auf Beschädigungen untersucht und bei einem Defekt ersetzt zu werden.

Auf dieser Grundlage schlägt die Erfindung vor, den Aerosolerzeuger mit einer Charakterisierungseinrichtung und die Inhalationstherapievorrichtung mit einer Erkennungseinrichtung auszustatten, die miteinander in Verbindung stehen, wenn der Aerosolerzeuger in die Inhalationsvorrichtung eingesetzt ist, damit der Aerosolerzeuger unmittelbar vor und während des Betriebs durch die Inhalationsvorrichtung überprüft und auf diese Weise die Funktionsfähigkeit und Dosisgenauigkeit der Inhalationstherapievorrichtung abgesichert werden kann.

Durch die Charakterisierungseinrichtung wird die Möglichkeit geschaffen, den Aerosolerzeuger zu individualisieren, d.h. mit Informationen auszustatten, die ihn gegenüber anderen Aerosolerzeugern gleicher oder anderer Bauart auszeichnen und unterscheidbar machen, die in einer vorteilhaften Ausgestaltung aber auch die Möglichkeit bieten, sich ändernde Eigenschaften/Parameter des Aerosolerzeugers anzugeben. Auf der Basis dieser Informationen kann in der Inhalationstherapievorrichtung beispielweise festgestellt werden, ob ein für die Inhalationstherapievorrichtung geeigneter Aerosolerzeuger eingesetzt wurde, ob der Aerosolerzeuger schon eine vorgegebene maximale Betriebsdauer erreicht hat oder ob bei der Fertigung ermittelte Eigenschaften/Parameter während der Ansteuerung zu berücksichtigen sind.

Weitere Anwendungsmöglichkeiten sowie Einzelheiten der Erfindung gehen aus der folgenden Schilderung von Ausführungsbeispielen der Erfindung hervor. Dabei wird auf die Zeichnungen Bezug genommen, in denen zeigt:
- Figur 1: ein Blockdiagramm mit einer schematischen Darstellung des Aufbaus einer erfindungsgemäßen Inhalationstherapievorrichtung;
- Figur 2: ein Blockdiagramm mit einer schematischen Darstellung eines ersten Ausführungsbeispiels einer Charakterisierungseinrichtung und einer Erkennungseinrichtung gemäß der Erfindung;
- Figur 3: ein Blockdiagramm mit einer schematischen Darstellung eines zweiten Ausführungsbeispiels einer Charakterisierungseinrichtung und einer Erkennungseinrichtung gemäß der Erfindung;
- Figur 4: ein Blockdiagramm mit einer schematischen Darstellung eines dritten Ausführungsbeispiels einer Charakterisierungseinrichtung und einer Erkennungseinrichtung gemäß der Erfindung; und
- Figur 5: ein Blockdiagramm mit einer schematischen Darstellung eines vierten Ausführungsbeispiels einer Charakterisierungseinrichtung und einer Erkennungseinrichtung gemäß der Erfindung.

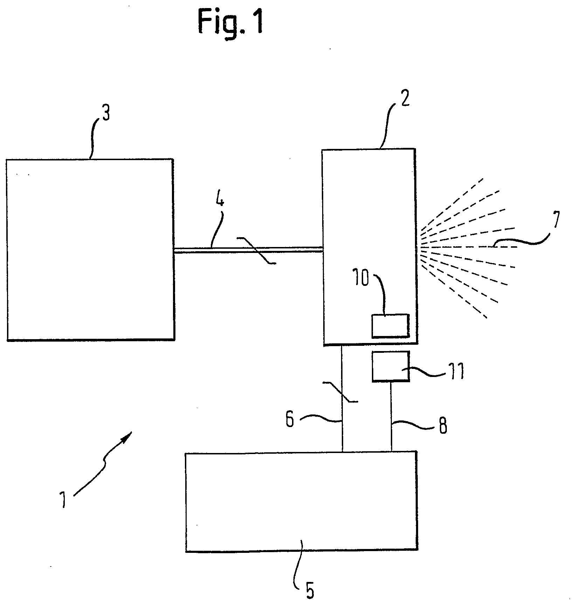

Figur 1 zeigt in einer schematischen Darstellung eine erfindungsgemäße Inhalationsvorrichtung 1, die einen Aerosolerzeuger 2 umfasst, der mit einem Flüssigkeitsreservoir 3 über eine Flüssigkeitsverbindung 4 derart in Verbindung steht, dass eine in dem Flüssigkeitsreservoir 3 bevorratete Flüssigkeit dem Aerosolerzeuger 2 zugeführt wird. Eine Ansteuereinrichtung 5 führt dem Aerosolerzeuger 2 über eine zumeist elektrische Verbindung 6 ein Ansteuerungssignal zu, das den Aerosolerzeuger 2 veranlasst, die Flüssigkeit aus dem Flüssigkeitsreservoir 3 zu vernebeln, so dass ein Aerosol 7 erzeugt wird. Das Aerosol 7 wird einem Patienten zur Inhalation dargeboten, wofür die Inhalationstherapievorrichtung 1 üblicherweise ein Mundstück (nicht dargestellt) oder dergleichen aufweist.

Der Aerosolerzeuger 2 ist aus der Inhalationstherapievorrichtung 1 entnehmbar bzw. in diese einsetzbar. Aus diesem Grund sind die Flüssigkeitsverbindung 4 und die Ansteuerverbindung 6 so ausgestaltet, dass beim Einsetzen des Aerosolerzeugers 2 in die Inhalationstherapievorrichtung 1 die jeweilige Verbindung hergestellt und beim Entnehmen gelöst wird.

Erfindungsgemäß umfasst der Aerosolerzeuger 2 eine Charakterisierungseinrichtung 10, in der zumindest solche Informationen I abgelegt sind, die den Aerosolerzeuger 2 individualisieren, d.h. die ihn gegenüber anderen Aerosolerzeugern gleicher oder anderer Bauart auszeichnen und ihn unterscheidbar machen. Quasi als Gegenstück umfasst die Inhalationstherapievorrichtung 1 eine Erkennungseinrichtung 11, die mit der Charakterisierungseinrichtung 10 zusammenwirkt, d.h. die zumindest auf die abgelegten Informationen zugreifen kann, um die den Aerosolgenerator charakterisierenden Informationen I zu ermitteln.

Die so ermittelten Informationen I über den Aerosolerzeuger 2 können auf verschiedene Weise genutzt werden. Beispielsweise kann die Erkennungseinrichtung 11 so ausgelegt sein, dass eine Überprüfung der Informationen bzw. ein Abgleich mit Referenzinformationen, die vorzugsweise in der Erkennungseinrichtung 11 oder in einem separaten Speicher abgespeichert sind, stattfindet und in Abhängigkeit von dem Ergebnis die Ansteuereinrichtung 5 aktiviert oder deaktiviert wird. Dazu führt die Erkennungseinrichtung 11 über die in Figur 1 gezeigte Signalverbindung 8 der Ansteuereinrichtung 5 ein Aktivierungs-/Deaktivierungssignal zu. Vorzugsweise gibt die Erkennungseinrichtung 11 aber die Informationen I an die Ansteuereinrichtung 5 über eine geeignet gestaltete, vorzugsweise elektrische Informationsverbindung 8 weiter.

Im Hinblick auf die Charakterisierungseinrichtung 10 und die Erkennungseinrichtung 11 bieten sich verschiedene Möglichkeiten der Ausgestaltung an.

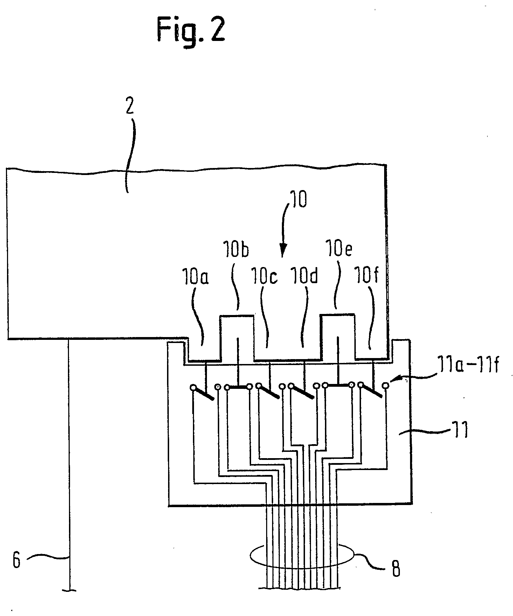

In einer ersten Ausgestaltung gemäß Figur 2, in der nur der in Figur 1 angedeutete Ausschnitt gezeigt ist, ist die Charakterisierungseinrichtung 10 in Form einer Mehrzahl von Vertiefungen und/oder Vorsprüngen 10a bis 10f realisiert, die an der äußeren Oberfläche des Aerosolerzeugers 2 vorgesehen sind und deren Anzahl bzw. Anordnung/Abfolge den Aerosolgenerator 2 kennzeichnen. Die Erkennungseinrichtung 11 ist dann beispielweise als eine Anordnung von einer entsprechenden Mehrzahl von Schaltern 11a bis 11f realisiert, wie Figur 2 beispielhaft zeigt, die der Anordnung von Vertiefungen und Vorsprüngen folgend geschaltet sind, wenn der Aerosolerzeuger in die Inhalationstherapievorrichtung 1 eingesetzt ist. Die Schalter 11a bis 11f sind über die Informationsverbindung 8 mit der Ansteuereinrichtung 5 verbunden, so dass die Information I dort verwertet werden kann.

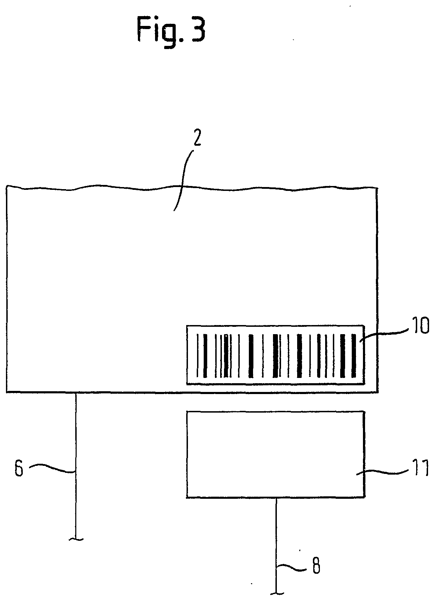

In einer zweiten Ausgestaltung gemäß Figur 3, in der nur der in Figur 1 angedeutete Ausschnitt gezeigt ist, ist die Charakterisierungseinrichtung 10 in Form eines Barcode-Aufklebers oder -Aufdrucks auf einer äußeren Oberfläche des Aerosolerzeugers 2 realisiert. Die Erkennungseinrichtung 11 ist dementsprechend ein Barcode-Leser, der so angeordnet ist, dass der Inhalt des Barcode-Aufklebers oder -Aufdrucks gelesen werden kann und die in dem Barcode abgelegten Informationen über die Informationsverbindung 8 der Ansteuereinrichtung 5 zur Verwertung zugeführt werden können. Anstelle des in der Figur 3 gezeigten Barcodes können auch andere maschinenlesbare Codes, z.B. Dot-Matrix-Codes oder 2D-Barcodes verwendet werden.

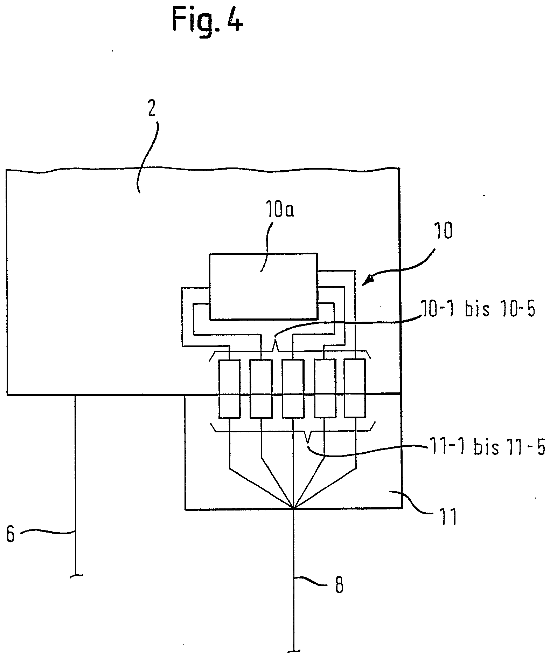

In einer dritten Ausgestaltung gemäß Figur 4, in der nur der in Figur 1 angedeutete Ausschnitt gezeigt ist, ist die Charakterisierungseinrichtung 10 in Form eines elektrischen Speichers 10a realisiert, der mit einer Mehrzahl erster Anschlüsse 10-1 bis 10-5 verbunden ist, die auf einer äußeren Oberfläche des Aerosolerzeugers 2 angeordnet sind. Die Erkennungseinrichtung 11 weist entsprechende Mehrzahl zweiter Anschlüsse 11-1 bis 11-5 auf, die die ersten Anschlüsse 10-1 bis 10-5 kontaktieren, wenn der Aerosolerzeuger 2 in die Inhalationsvorrichtung 1 eingesetzt ist.

Als Speicher 10a eignet sich jeder nicht-flüchtige Speicher, wie zum Beispiel ein EPROM, der bei der Herstellung des Aerosolerzeugers 2 programmiert wird und dann ohne Energieversorgung die programmierten Inhalte speichert. Wenn gemäß einer bevorzugten Ausgestaltung ein nicht-flüchtiger Schreib-Lese-Speicher eingesetzt wird, beispielsweise ein EEPROM oder ein sogenannter Flash-Speicher, können nicht nur bei der Herstellung auf einfache Weise die gewünschten Informationen zum Aerosolerzeuger 2 in dem Speicher 10a abgelegt werden, sondern auch Informationen, die während des Betriebs der Inhalationstherapievorrichtung 1 anfallen, in den Speicher des Aerosolerzeugers 2 gespeichert werden. Dazu ist vorzugsweise die Ansteuereinheit 2 mit einer Programmierfunktion ausgestattet.

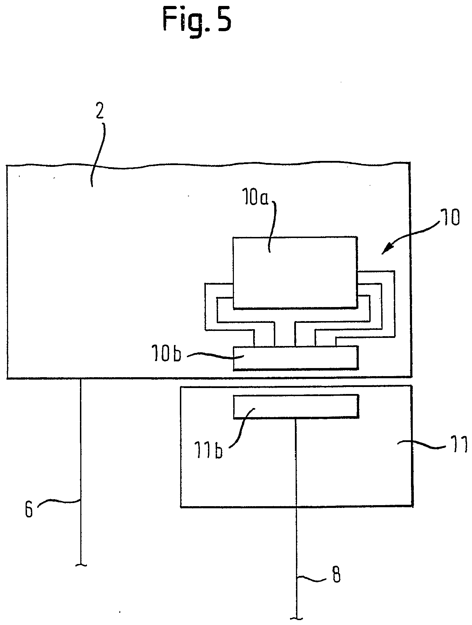

Gemäß einer vierten Ausgestaltung, die in Figur 5 gezeigt ist, die nur den in Figur 1 angedeuteten Ausschnitt wiedergibt, ist der Speicher 10a nicht mit Anschlusskontakten, sondern mit einer kontaktlosen ersten Verbindungseinrichtung 10b verbunden. Die Erkennungseinrichtung 11 umfasst eine entsprechende kontaktlose zweite Verbindungseinrichtung 11b, die eine Verbindung zu der ersten Verbindungseinrichtung 10b gewährleistet, wenn der Aerosolerzeuger 2 in die Inhalationstherapievorrichtung 1 eingesetzt ist. Der Inhalt des Speichers 10a wird dann von der Erkennungseinrichtung 11 über die mit Hilfe der beiden kontaktlosen Verbindungseinrichtungen 10b und 11b hergestellte Verbindung ausgelesen und die so ermittelnden Informationen über den Aerosolerzeuger 2 von der Erkennungseinrichtung 11 an die Ansteuereinrichtung 5 weitergegeben. Die kontaktlose Verbindung kann mittels Funk oder Licht, vorzugsweise Infrarotlicht, oder auf magnetischem Weg bewerkstelligt werden.

Die in der Charakterisierungseinrichtung 10 abgelegten Informationen bezüglich des Aerosolerzeugers 2 können sich auf verschiedenste Aspekte des Aerosolerzeugers 2 beziehen und auf unterschiedliche Weise in den Betrieb des Aerosolerzeugers bzw. der Inhalationstherapievorrichtung einbezogen werden. In der Regel übernimmt die Ansteuereinrichtung 5 die Aufgabe der Einbeziehung der ermittelten Informationen in die Ansteuerung des Aerosolgenerators 2 und - sofern vorgesehen - des Ablegens von Informationen in der Charakterisierungseinrichtung, die bei der Fertigung des Aerosolerzeugers 2 noch nicht vorlagen, sondern erst während des Betriebs der Inhalationstherapievorrichtung 1 entstehen.

Bei den in der Charakterisierungseinrichtung 10 eines erfindungsgemäßen Aerosolerzeugers 2 abgelegten Informationen handelt es sich beispielsweise um folgende bei der Fertigung ablegbare Informationen:
- Typangaben, die eine Typbezeichnung bzw. Betriebsparameter (z.B. Ansteuerspannung, Resonanzfrequenz etc.) des Aerosolerzeugers umfassen;
- Fertigungsinformationen wie das Fertigungsdatum und/oder eine Chargenbezeichnung;
- eine maximale Verwendungsdauer;
- eine maximale Betriebsdauer;
- eine maximale Verwendungsanzahl/-häufigkeit;
- zulässige Therapieanwendungen.

Sofern Informationen während des Betriebs der Inhalationstherapievorrichtung 1 in der Charakterisierungseinrichtung 10 ablegbar sind, können ferner folgende Informationen vorgesehen werden:
- Erstverwendungsdatum;
- tatsächliche Betriebsdauer;
- tatsächliche Verwendungsanzahl/-häufigkeit.

In einer erfindungsgemäßen Inhalationstherapievorrichtung 1 ist die Ansteuereinrichtung 5 vorteilhafterweise in einer programmgesteuerten Form realisiert, so dass eine Einbeziehung der aus der Charakterisierungseinrichtung ermittelten Informationen in besonderem Maße unproblematisch umsetzbar ist. Die Verwendung einiger der oben angegebenen Informationen bei der Ansteuerung des Aerosolerzeugers 2 wird im Folgenden beispielhaft beschrieben, wobei erkennbar ist, dass damit jeweils eine Absicherung des Betriebs der erfindungsgemäßen Inhalationstherapievorrichtung einhergeht.

Die Ansteuereinrichtung 5 kann so ausgelegt sein, dass anhand der ermittelten Typbezeichnung festgestellt wird, ob der eingesetzte Aerosolerzeuger 2 für die Verwendung mit der Inhalationstherapievorrichtung 1 geeignet/zugelassen ist. Auf diesem Weg wird sichergestellt, dass kein ungeeigneter Aerosolerzeuger in der Inhalationstherapievorrichtung verwendet wird.

Durch die Einbeziehung von ermittelten Betriebsparametern kann beispielsweise gewährleistet werden, dass die Ansteuereinrichtung 5 ein Ansteuerungssignal an den Aerosolerzeuger 2 abgibt, das die richtigen Parameter, zum Beispiel die richtige Spannung und die richtige Frequenz aufweist. Dadurch wird eine Verwendung der Inhalationstherapievorrichtung mit einem in dieser Hinsicht falsch angesteuerten Aerosolerzeuger verhindert, gleichzeitig aber die Möglichkeit geschaffen, dass unterschiedliche Aerosolerzeuger in der Inhalationstherapievorrichtung verwendet werden.

Die Ansteuereinrichtung 5 kann unter Bezugnahme auf ein aktuelles Tagesdatum, das zum Beispiel von einem in die Ansteuereinrichtung integrierten Zeitgeber bereitgestellt wird, anhand des ermittelten Fertigungsdatums feststellen, ob der Aerosolerzeuger 2 noch betrieben werden darf. Auf ähnliche Weise kann die Ansteuervorrichtung 5 feststellen, ob eine maximale Verwendungsdauer, eine maximale Betriebsdauer oder eine maximale Verwendungsanzahl/- häufigkeit noch nicht erreicht ist. Damit wird sichergestellt, dass der Aerosolerzeuger nicht mehr verwendet wird, wenn zu befürchten ist, dass aufgrund der langen Zeiträume oder der häufigen Verwendung unzuverlässige Verneblungsergebnisse erzielt werden, die die Dosisgenauigkeit der Inhalationstherapievorrichtung beeinträchtigen.

Eine erfindungsgemäße Inhalationstherapievorrichtung zeichnet sich trotz der hohen Absicherung der Funktionsfähigkeit und Dosisgenauigkeit durch eine großes Maß an Flexibilität aus. Denn die Ansteuerung des Aerosolerzeugers 2 kann in Abhängigkeit vom ermittelten Typ auf unterschiedliche Art, beispielsweise unter Zugrundelegung unterschiedlicher Ansteuerungssignale erfolgen. Dazu verfügt die Ansteuereinrichtung 2 über verschiedene Ansteuerschemata, die jeweils einem oder mehreren Aerosolerzeugertypen zugeordnet sind, so dass die Ansteuerung entsprechend dem zugeordneten bzw. einem aus den zugeordneten Schemata ausgewählten Schema erfolgt. In den Ansteuerungsschemata sind beispielweise Resonanzfrequenzen, Einschaltzeitpunkte/-verzögerungen, Ausschaltzeitpunkte/-verzögerungen und/oder Signalverläufe festgelegt, die bei der Erzeugung des Ansteuerungssignals zugrunde gelegt werden. Die Ansteuerungsschemata/-programme sind vorzugsweise in einem Speicher der Ansteuereinrichtung 5 abgespeichert, der als Daten-/Parameterspeicher, in dem Daten für die Erzeugung des Ansteuerungssignals abgelegt sind, oder als Programmspeicher ausgelegt sein kann, in dem unterschiedliche Programme für die Erzeugung des Ansteuerungssignals abgespeichert sind, die in Abhängigkeit von den den Aerosolerzeuger charakterisierenden Informationen ausgewählt und abgearbeitet werden.

Der Betrieb einer erfindungsgemäßen Inhalationstherapievorrichtung 1 zeichnet sich aus durch den Schritt des Ermittelns von den Aerosolerzeuger 2 charakterisierenden Informationen I, d.h. dadurch dass die Erkennungseinrichtung 11 die Informationen aus der Charakterisierungseinrichtung 10 abruft. In einer besonderen Ausgestaltung wird auf der Basis von zumindest einem Teil der Informationen die Ansteuereinrichtung 5 aktiviert bzw. Deaktiviert wie weiter oben beschrieben wurde. In einer speziellen Ausgestaltung wird überprüft, ob die Verwendung des Aerosolerzeugers in der Inhalationstherapievorrichtung zulässig ist oder ob eine maximale Verwendung des Aerosolerzeugers erreicht ist. In einer weiteren Ausgestaltung wird auf der Basis von zumindest einem Teil der Informationen ein Ansteuerschema/- programm für die Erzeugung des Ansteuersignals ausgewählt.

## Patentansprüche

1. Inhalationstherapievorrichtung (1)
- mit einem Aerosolerzeuger (2), der in die Inhalationstherapievorrichtung einsetzbar bzw. aus der Inhalationstherapievorrichtung entnehmbar ist und dem zur Aerosolerzeugung ein Ansteuersignal zuführbar ist, und
- mit einer Ansteuereinrichtung (5) für die Bereitstellung des Ansteuerungssignals, das dem in die Inhalationsvorrichtung eingesetzten Aerosolerzeuger während der Aerosolerzeugung zugeführt wird,
wobei
- der Aerosolerzeuger (2) eine Charakterisierungseinrichtung (10) für die Bereitstellung von den Aerosolerzeuger charakterisierenden Informationen umfasst und
- die Inhalationsvorrichtung (1) eine Erkennungseinrichtung (11) aufweist, die mit der Charakterisierungseinrichtung eines in die Inhalationstherapievorrichtung eingesetzten Aerosolerzeugers zum Ermitteln der den Aerosolerzeuger charakterisierenden Informationen (I) zusammenwirkt,
wobei
die charakterisierende Information (I) Typangaben, die eine Typbezeichnung und/oder Betriebsparameter des Aerosolerzeugers umfasst, und wobei
die Ansteuereinrichtung (5) ausgelegt ist, anhand der ermittelten Typbezeichnung in den charakterisierenden Informationen (I) festzustellen, ob der eingesetzte Aerosolerzeuger (2) für die Inhalationstherapievorrichtung (1) geeignet/zugelassen ist.

2. Inhalationstherapievorrichtung nach Anspruch 1, **dadurch gekennzeichnet, dass** die Erkennungseinrichtung (11) auf der Basis der ermittelten den Aerosolerzeuger charakterisierenden Informationen (I) die Ansteuereinrichtung (5) aktiviert oder deaktiviert, indem die Erkennungseinrichtung (11) der Ansteuereinrichtung (5) ein Aktivierungs- bzw. ein Deaktivierungssignal zuführt.

3. Inhalationstherapievorrichtung nach Anspruch 1, **dadurch gekennzeichnet, dass** die Erkennungseinrichtung (11) die ermittelten den Aerosol charakterisierenden Informationen der Ansteuereinrichtung (5) zuführt.

4. Inhalationstherapievorrichtung nach einem der Ansprüche 1 bis 3, **dadurch gekennzeichnet, dass** die Charakterisierungseinrichtung (10) eine Mehrzahl von Vertiefungen/Vorsprüngen (10a bis 10e) auf einer Oberfläche des Aerosolerzeugers (2) und die Erkennungseinrichtung (11) eine Anordnung von einer entsprechenden Mehrzahl von Schaltern (11a bis 11e) umfasst, deren Schaltstellungen von den Vertiefungen/Vorsprüngen (10a bis 10e) beeinflusst werden, wenn der Aerosolerzeuger (2) in die Inhalationstherapievorrichtung (1) eingesetzt ist.

5. Inhalationstherapievorrichtung nach einem der Ansprüche 1 bis 4, **dadurch gekennzeichnet, dass** die Charakterisierungseinrichtung (10) einen Barcode-Aufkleber oder -Aufdruck auf einer Oberfläche des Aerosolerzeugers (2) und die Erkennungseinrichtung (11) einen Barcode-Leser umfasst.

6. Inhalationstherapievorrichtung nach einem der Ansprüche 1 bis 5, **dadurch gekennzeichnet, dass** die Charakterisierungseinrichtung (10) einen elektrischen Speicher (10a) umfasst.

7. Inhalationstherapievorrichtung nach Anspruch 6, **dadurch gekennzeichnet, dass** der Speicher (10a) mit einer Mehrzahl erster Anschlüsse (10-1 bis 10-5) verbunden ist, die mit jeweils einem Anschluss einer Mehrzahl zweiter Anschlüsse (11-1 bis 11-5) in Kontakt stehen, wenn der Aerosolerzeuger (2) in die Inhalationstherapievorrichtung (1) eingesetzt ist.

8. Inhalationstherapievorrichtung nach Anspruch 6, **dadurch gekennzeichnet, dass** der Speicher (10a) mittels einer kontaktlosen ersten Verbindungseinrichtung (10b) mit einer entsprechenden kontaktlosen zweiten Verbindungseinrichtung (11b) der Erkennungseinrichtung (11) verbunden ist.

9. Inhalationstherapievorrichtung nach einem der Ansprüche 6 bis 8, **dadurch gekennzeichnet, dass** der Speicher (10a) ein nicht-flüchtiger Speicher, insbesondere ein EPROM ist.

10. Inhalationstherapievorrichtung nach einem der Ansprüche 6 bis 9, **dadurch gekennzeichnet, dass** der Speicher (10a) ein nicht-flüchtiger Schreib-Lese-Speicher, insbesondere ein EEPROM oder ein Flash-Speicher ist.

11. Inhalationstherapievorrichtung nach einem der vorangegangenen Ansprüche, **dadurch gekennzeichnet, dass** die den Aerosolerzeuger charakterisierenden Informationen (I) weiter eine oder mehrere der folgenden Angaben umfassen:
- Fertigungsinformationen wie das Fertigungsdatum und/oder eine Chargenbezeichnung;
- eine maximale Verwendungsdauer;
- eine maximale Betriebsdauer;
- eine maximale Verwendungsanzahl/-häufigkeit;
- zulässige Therapieanwendungen;
- Erstverwendungsdatum;
- tatsächliche Betriebsdauer;
- tatsächliche Verwendungsanzahl/-häufigkeit.

12. Inhalationstherapievorrichtung nach einem der vorangegangenen Ansprüche, **dadurch gekennzeichnet, dass** die Ansteuereinrichtung (5) ausgelegt ist, anhand der ermittelten Informationen (I) zu gewährleisten, dass dem Aerosolerzeuger (2) ein Ansteuerungssignal mit richtigen Parametern, wie Spannung und Frequenz zugeführt wird.

13. Inhalationstherapievorrichtung nach einem der vorangegangenen Ansprüche, **dadurch gekennzeichnet, dass** die Ansteuereinrichtung (5) ausgelegt ist, anhand der ermittelten Informationen (I) festzustellen, ob der Aerosolerzeuger (2) vor dem Hintergrund eines zu langen oder zu häufigen Einsatzes noch betrieben werden darf.

14. Inhalationstherapievorrichtung nach einem der vorangegangenen Ansprüche, **dadurch gekennzeichnet, dass** die Ansteuereinrichtung (5) ausgelegt ist, anhand der ermittelten Informationen (I) festzustellen, welches Anwendungsschema/-programm für die Erzeugung des Ansteuerungssignals für den Aerosolerzeuger (2) zugrunde zu legen ist.

15. Inhalationstherapievorrichtung nach Anspruch 14, **dadurch gekennzeichnet, dass** die Ansteuereinrichtung (5) einen Speicher für Ansteuerschemata/-programme aufweist.

16. Inhalationstherapievorrichtung nach einem der vorangegangenen Ansprüche, **dadurch gekennzeichnet, dass** die Ansteuereinrichtung (5) ausgelegt ist, um Informationen in der Charakterisierungseinrichtung (10) abzulegen.

17. Inhalationstherapievorrichtung nach einem der vorangegangenen Ansprüche, **dadurch gekennzeichnet, dass** der Aerosolerzeuger (2) eine Membran und eine Schwingungserzeugungseinrichtung aufweist.

18. Inhalationstherapievorrichtung nach Anspruch 17, **dadurch gekennzeichnet, dass** eine in einem Flüssigkeitsreservoir bevorratete Flüssigkeit einer Seite der Membran zugeführt wird.

19. Verfahren zur Erzeugung eines Aerosol mit einer Inhalationstherapievorrichtung (1) mit den Merkmalen eines der vorangegangenen Ansprüche, **gekennzeichnet durch** den Schritt des Ermittelns von den Aerosolerzeuger (2) charakterisierenden Informationen (I).

20. Verfahren nach Anspruch 19, **gekennzeichnet durch** den Schritt des Untersuchens zumindest eines Teils der ermittelten Informationen (I) und den Schritt des Aktivierens bzw. Deaktivierens der Ansteuereinrichtung (5) .

21. Verfahren nach Anspruch 19, **gekennzeichnet durch** den Schritt des Untersuchens zumindest eines Teils der ermittelten Informationen (I) und den Schritt des Überprüfens, ob die Verwendung des Aerosolerzeugers (2) in der Inhalationstherapievorrichtung (1) zulässig ist.

22. Verfahren nach Anspruch 19, 20 oder 21, **gekennzeichnet durch** den Schritt des Untersuchens zumindest eines Teils der ermittelten Informationen (I) und den Schritt des Überprüfens, ob eine maximale Verwendung des Aerosolerzeugers (2) erreicht ist.

23. Verfahren nach Anspruch 19, 20, 21 oder 22, **gekennzeichnet durch** den Schritt des Untersuchens zumindest eines Teils der ermittelten Informationen (I) und den Schritt der Auswahl eine Ansteuerungsschemas/- programms.

## Claims

1. An inhalation therapy device (1)
- having an aerosol generator (2), which can be inserted into the inhalation therapy device or removed from the inhalation therapy device and to which a control signal can be supplied for aerosol generation, and
- having a control device (5) for providing the control signal that is supplied to the aerosol generator inserted in the inhalation therapy device during aerosol generation,
- the aerosol generator (2) comprising a characterization device (10) for the provision of information characterizing the aerosol generator, and
- the inhalation device (1) comprising an identification device (11), which cooperates with the characterization device of an aerosol generator inserted in the inhalation therapy device for determining the information (I) characterizing the aerosol generator,
the characterizing information (I) comprising type details which include a type designation and/or operating parameters of the aerosol generator, and
the control device (5) being designed to ascertain, on the basis of the type designation determined in the characterizing information (I), whether the inserted aerosol generator (2) is suitable/permissible for the inhalation therapy device (1).

2. The inhalation therapy device according to Claim 1, **characterized in that** the identification device (11) activates or deactivates the control device (5) based on the determined information (I) characterizing the aerosol generator, **in that** the identification device (11) supplies an activation or deactivation signal to the control device (5).

3. The inhalation therapy device according to Claim 1, **characterized in that** the identification device (11) supplies the determined information characterizing the aerosol to the control device (5).

4. The inhalation therapy device according to one of Claims 1 to 3, **characterized in that** the characterization device (10) comprises a plurality of recesses/projections (10a to 10e) on a surface of the aerosol generator (2) and the identification device (11) comprises an arrangement of a corresponding number of switches (11a to 11e), the switch positions of which are influenced by the recesses/projections (10a to 10e) when the aerosol generator (2) is inserted in the inhalation therapy device (1).

5. The inhalation therapy device according to one of Claims 1 to 4, **characterized in that** the characterization device (10) comprises a barcode sticker or barcode imprint on a surface of the aerosol generator (2) and the identification device (11) comprises a barcode reader.

6. The inhalation therapy device according to one of Claims 1 to 5, **characterized in that** the characterization device (10) comprises an electrical memory (10a).

7. The inhalation therapy device according to Claim 6, **characterized in that** the memory (10a) is connected to a plurality of first terminals (10-1 to 10-5) which are each in contact with a terminal of a plurality of second terminals (11-1 to 11-5) when the aerosol generator (2) is inserted into the inhalation therapy device (1).

8. The inhalation therapy device according to Claim 6, **characterized in that** the memory (10a) is connected by means of a contactless first connecting device (10b) to a corresponding contactless second connecting device (11b) of the identification device (11).

9. The inhalation therapy device according to one of Claims 6 to 8, **characterized in that** the memory (10a) is a non-volatile memory, in particular an EPROM.

10. The inhalation therapy device according to one of Claims 6 to 9, **characterized in that** the memory (10a) is a non-volatile read/write memory, in particular an EEPROM or a flash memory.

11. The inhalation therapy device according to one of the preceding claims, **characterized in that** the information (I) characterizing the aerosol generator also includes one or more of the following details:
- manufacturing information such as the date of manufacture and/or a batch identifier;
- a maximum period of use;
- a maximum service life;
- a maximum number/frequency of use;
- permissible therapy applications;
- date of first use;
- actual service life;
- actual number/frequency of use.

12. The inhalation therapy device according to one of the preceding claims, **characterized in that** the control device (5) is designed to ensure, on the basis of the information (I) determined, that a control signal with correct parameters, such as voltage and frequency, is supplied to the aerosol generator (2).

13. The inhalation therapy device according to one of the preceding claims, **characterized in that** the control device (5) is designed to ascertain, on the basis of the information (I) determined, whether the aerosol generator (2) may still be operated against the background of too long or too frequent a use.

14. The inhalation therapy device according to one of the preceding claims, **characterized in that** the control device (5) is designed to ascertain, on the basis of the information (I) determined, which application scheme/program is to be used as the basis for generating the control signal for the aerosol generator (2).

15. The inhalation therapy device according to Claim 14, **characterized in that** the control device (5) has a memory for control schemes/programs.

16. The inhalation therapy device according to one of the preceding claims, **characterized in that** the control device (5) is designed to store information in the characterization device (10).

17. The inhalation therapy device according to one of the preceding claims, **characterized in that** the aerosol generator (2) has a membrane and an oscillation generating device.

18. The inhalation therapy device according to Claim 17, **characterized in that** a liquid stored in a liquid reservoir is supplied to one side of the membrane.

19. A method for the generation of an aerosol using an inhalation therapy device (1) having the features of one of the preceding claims, **characterized by** the step of determining the information (I) characterizing the aerosol generator (2).

20. The method according to Claim 19, **characterized by** the step of examining at least a part of the information (I) determined and the step of activating or deactivating the control device (5).

21. The method according to Claim 19, **characterized by** the step of examining at least a part of the information (I) determined and the step of checking whether the use of the aerosol generator (2) in the inhalation therapy device (1) is permissible.

22. The method according to Claim 19, 20 or 21, **characterized by** the step of examining at least a part of the information (I) determined and the step of checking whether a maximum use of the aerosol generator (2) has been reached.

23. The method according to Claim 19, 20, 21 or 22, **characterized by** the step of examining at least a part of the information (I) determined and the step of selecting a control diagram/program.

## Revendications

1. Dispositif de thérapie par inhalation (1),
- avec un générateur d'aérosol (2), qui peut être inséré dans le dispositif de thérapie par inhalation ou peut être enlevé du dispositif de thérapie par inhalation et auquel un signal de commande peut être envoyé afin de produire un aérosol, et
- avec un dispositif de commande (5) pour la fourniture du signal de commande qui est envoyé pendant la production d'aérosol au générateur d'aérosol inséré dans le dispositif d'inhalation,
dans lequel :
- le générateur d'aérosol (2) comprend un dispositif de caractérisation (10) pour la fourniture d'informations caractérisant le générateur d'aérosol, et
- le dispositif d'inhalation (1) comporte un dispositif de reconnaissance (11) qui coopère avec le dispositif de caractérisation d'un générateur d'aérosol inséré dans le dispositif de thérapie par inhalation afin de déterminer les informations (I) caractérisant le générateur d'aérosol,
dans lequel :
- l'information caractérisante (I) comprend des indications de type, qui comprennent une désignation de type et/ou des paramètres de fonctionnement du générateur d'aérosol, et dans lequel :
- le dispositif de commande (5) est conçu pour déterminer, à l'aide de la désignation de type déterminée dans les informations caractérisantes (I), si le générateur d'aérosol (2) inséré est approprié/autorisé pour le dispositif de thérapie par inhalation (1).

2. Dispositif de thérapie par inhalation selon la revendication 1, **caractérisé en ce que** le dispositif de reconnaissance (11) active ou désactive le dispositif de commande (5) sur la base des informations (I) déterminées caractérisant le générateur d'aérosol, le dispositif de reconnaissance (11) envoyant à cet effet au dispositif de commande (5) un signal d'activation ou de désactivation.

3. Dispositif de thérapie par inhalation selon la revendication 1, **caractérisé en ce que** le dispositif de reconnaissance (11) envoie les informations déterminées caractérisant l'aérosol au dispositif de commande (5).

4. Dispositif de thérapie par inhalation selon l'une quelconque des revendications 1 à 3, **caractérisé en ce que** le dispositif de caractérisation (10) comprend une pluralité de creux/bosses (10a à 10e) sur une surface du générateur d'aérosol (2) et le dispositif de reconnaissance (11) comprend un agencement d'une pluralité correspondante de commutateurs (11a à 11e) dont les positions de commutation sont influencées par les creux/bosses (10a à 10e) lorsque le générateur d'aérosol (2) est inséré dans le dispositif de thérapie par inhalation (1).

5. Dispositif de thérapie par inhalation selon l'une quelconque des revendications 1 à 4, **caractérisé en ce que** le dispositif de caractérisation (10) comprend un autocollant ou une surimpression à code-barres sur une surface du générateur d'aérosol (2) et le dispositif de reconnaissance (11) un lecteur de code-barres.

6. Dispositif de thérapie par inhalation selon l'une quelconque des revendications 1 à 5, **caractérisé en ce que** le dispositif de caractérisation (10) comprend une mémoire électrique (10a).

7. Dispositif de thérapie par inhalation selon la revendication 6, **caractérisé en ce que** la mémoire (10a) est reliée à une pluralité de premières bornes (10-1 à 10-5) qui sont en contact chacune avec une borne d'une pluralité de secondes bornes (11-1 à 11-5), lorsque le générateur d'aérosol (2) est inséré dans le dispositif de thérapie par inhalation (1).

8. Dispositif de thérapie par inhalation selon la revendication 6, **caractérisé en ce que** la mémoire (10a) est reliée au moyen d'un premier dispositif de liaison sans contact (10b) à un second dispositif de liaison sans contact (11b) correspondant du dispositif de reconnaissance (11).

9. Dispositif de thérapie par inhalation selon l'une quelconque des revendications 6 à 8, **caractérisé en ce que** la mémoire (10a) est une mémoire non volatile, en particulier une EPROM.

10. Dispositif de thérapie par inhalation selon l'une quelconque des revendications 6 à 9, **caractérisé en ce que** la mémoire (10a) est une mémoire d'écriture et de lecture non volatile, en particulier une EEPROM ou une mémoire flash.

11. Dispositif de thérapie par inhalation selon l'une quelconque des revendications précédentes, **caractérisé en ce que** les informations (I) caractérisant le générateur d'aérosol comprennent en outre une ou plusieurs des indications suivantes :
- des informations de fabrication telles que la date de fabrication et/ou une désignation de lot ;
- une durée d'utilisation maximale ;
- une durée de fonctionnement maximale ;
- un nombre ou une fréquence d'utilisation maximal(e) ;
- des applications thérapeutiques autorisées ;
- une date de première utilisation ;
- une durée de fonctionnement réelle ;
- un nombre ou une fréquence d'utilisation réel(le).

12. Dispositif de thérapie par inhalation selon l'une quelconque des revendications précédentes, **caractérisé en ce que** le dispositif de commande (5) est conçu pour garantir à l'aide des informations (I) déterminées qu'un signal de commande avec des paramètres corrects, tels que la tension et la fréquence, est envoyé au générateur d'aérosol (2).

13. Dispositif de thérapie par inhalation selon l'une quelconque des revendications précédentes, **caractérisé en ce que** le dispositif de commande (5) est conçu pour déterminer à l'aide des informations (I) déterminées si, en raison d'une utilisation trop longue ou trop fréquente, le générateur d'aérosol (2) peut encore être utilisé.

14. Dispositif de thérapie par inhalation selon l'une quelconque des revendications précédentes, **caractérisé en ce que** le dispositif de commande (5) est conçu pour déterminer à l'aide des informations (I) déterminées quel schéma/programme d'application doit être choisi pour la production du signal de commande destiné au générateur d'aérosol (2).

15. Dispositif de thérapie par inhalation selon la revendication 14, **caractérisé en ce que** le dispositif de commande (5) comporte une mémoire destinée à des schémas/programmes de commande.

16. Dispositif de thérapie par inhalation selon l'une quelconque des revendications précédentes, **caractérisé en ce que** le dispositif de commande (5) est conçu pour enregistrer des informations dans le dispositif de caractérisation (10).

17. Dispositif de thérapie par inhalation selon l'une quelconque des revendications précédentes, **caractérisé en ce que** le générateur d'aérosol (2) comporte une membrane et un dispositif de production d'oscillations.

18. Dispositif de thérapie par inhalation selon la revendication 17, **caractérisé en ce qu'**un liquide stocké dans un réservoir de liquide est envoyé à un côté de la membrane.

19. Procédé de production d'un aérosol avec un dispositif de thérapie par inhalation (1) ayant les caractéristiques de l'une quelconque des revendications précédentes, **caractérisé par** l'étape de la détermination d'informations (I) caractérisant le générateur d'aérosol (2).

20. Procédé selon la revendication 19, **caractérisé par** l'étape de l'examen d'au moins une partie des informations (I) déterminées et par l'étape de l'activation ou de la désactivation du dispositif de commande (5).

21. Procédé selon la revendication 19, **caractérisé par** l'étape de l'examen d'au moins une partie des informations (I) déterminées et par l'étape visant à vérifier si l'utilisation du générateur d'aérosol (2) dans le dispositif de thérapie par inhalation (1) est autorisée.

22. Procédé selon la revendication 19, 20 ou 21, **caractérisé par** l'étape de l'examen d'au moins une partie des informations (I) déterminées et par l'étape visant à vérifier si une utilisation maximale du générateur d'aérosol (2) est atteinte.

23. Procédé selon la revendication 19, 20, 21 ou 22, **caractérisé par** l'étape de l'examen d'au moins une partie des informations (I) déterminées et par l'étape de la sélection d'un schéma/programme de commande.
